# EUROPÄISCHE PATENTSCHRIFT

(11) **EP 3 413 713 B1**
(45) Veröffentlichungstag und Bekanntmachung des Hinweises auf die Patenterteilung: **16.08.2023**
(21) Anmeldenummer: 17709356.4
(22) Anmeldetag: 10.02.2017
(51) Int. Cl.: A01N 37/16, A01N 25/22, A01P 1/00, A61L 2/18

(54) **DESINFEKTIONSMITTEL**
DISINFECTANT AGENT
MOYEN DE DÉSINFECTION

(30) Priorität: 12.02.2016 DE 102016102485
(43) Veröffentlichungstag der Anmeldung: 19.12.2018
(73) Patentinhaber: Dantor Biotech AG, 6331 Hünenberg (CH)
(72) Erfinder: REICHWAGEN, Sven, 27476 Cuxhaven (DE)
(74) Vertreter: Schneiders & Behrendt Bochum
(86) Internationale Anmeldenummer: PCT/EP2017/052980
(87) Internationale Veröffentlichungsnummer: WO 2017/137546

(56) Entgegenhaltungen:
- WO-A1-92/19230
- WO-A1-2006/076334
- WO-A1-2011/005270
- WO-A2-2006/125657
- US-A- 5 008 106
- US-A1- 2014 004 208
- US-A1- 2014 121 271
- VIMONT ALLISON ET AL: "Study of the Virucidal Potential of Organic Peroxyacids Against Norovirus on Food-Contact Surfaces", FOOD AND ENVIRONMENTAL VIROLOGY, SPRINGER US, BOSTON, Bd. 7, Nr. 1, 22. November 2014 (2014-11-22), Seiten 49-57, XP035442936, ISSN: 1867-0334, DOI: 10.1007/S12560-014-9174-0 [gefunden am 2014-11-22]

## Beschreibung

Die Erfindung betrifft ein Mittel zum Desinfizieren von Oberflächen und Zerstören von bakteriellen Stoffwechselprodukten, das wenigstens eine Hydroxypercarbonsäure zusammen mit einem Stabilisierungsmittel in wässriger Lösung enthält.

Die Verwendung von Per Carbonsäuren zu Desinfektionszwecken ist grundsätzlich bekannt, beispielsweise aus der EP 0 677 990 B1. Insbesondere ist dort die Verwendung von Peressigsäure beschrieben. Das Desinfektionsmittel eignet sich unter anderem zur Behandlung von medizinischen Ausrüstungsgegenständen, beispielsweise Endoskopen.

Die Herstellung von Percarbonsäurelösungen erfolgt durch Umsetzung der Carbonsäure mit Wasserstoffperoxid in wässrigem Medium in Anwesenheit eines Katalysators, üblicherweise Schwefelsäure. Bei der Reaktion bildet sich ein Gleichgewicht zwischen der Percarbonsäure als Reaktionsprodukt einerseits und den Edukten, d.h. der Carbonsäure und Wasserstoffperoxid, andererseits aus. Häufig wird nach erfolgter Reaktion das Reaktionsgemisch mit einem Lösemittel weiter verdünnt, um die gewünschte niedrigere Percarbonsäurekonzentration einzustellen. So können etwa für Toilettenreinigungszwecke Lösungen eingesetzt werden, die 0,5 bis 1 Gew.-% Peressigsäure enthalten. In der Regel wird allerdings zunächst die Percarbonsäure in höher konzentrierter Lösung hergestellt, da andernfalls die Gleichgewichtseinstellung einen zu langen Zeitraum in Anspruch nehmen würde.

Auch bei Verdünnung einer konzentrierten Gleichgewichtslösung der Percarbonsäure mit Wasser verändert sich der Gleichgewichtspunkt des Systems zugunsten der Regenerierung der ursprünglichen Reaktanten, wobei auch diese Gleichgewichtseinstellung einen langen Zeitraum von mehreren Wochen oder Monaten in Anspruch nehmen kann. Entsprechend handelt es sich bei der verdünnten Percarbonsäurelösung um eine Lösung veränderlicher Zusammensetzung, die aufgrund der Zersetzung von Wasserstoffperoxid mehr und mehr ihre Wirkung verliert.

Aus der WO 2006/125 657 A2, auf deren Inhalt ausdrücklich Bezug genommen wird, ist ein wässriges Desinfektions-und Entkeimungsmittel bekannt, bei dem Percarbonsäuren durch den Zusatz von Sorbitanestern stabilisiert sind. Diese Methode hat sich als grundsätzlich erfolgreich erwiesen. Die Verwendung von Sorbitanestern schränkt aber den Einsatzbereich derartig stabilisierter Percarbonsäuren, etwa bei Lebensmitteln und im medizinischen und Pflegebereich, ein.

Ausgehend von diesem Stand der Technik stellt sich die Aufgabe, ein alternatives Stabilisierungsmitteln für Percarbonsäuren bereitzustellen, das die Anwendung damit stabilisierter Desinfektionsmittel in weiten Bereichen, insbesondere auch in der Lebensmittelindustrie und im medizinischen und Pflegebereich, ermöglicht. Ein solches Stabilisierungsmittel sollte physiologisch unbedenklich, gut hautverträglich sein und Percarbonsäuren für eine nachhaltige Wirkung hinreichend lang auf der behandelten Oberfläche stabilisieren.

Diese Aufgabe wird mit einem Mittel der eingangs genannten Art gelöst, wobei das Stabilisierungsmittel Polyvinylpyrrolidon (PVP) ist und das Mittel eine Hydroxypercarbonsäure enthält.

Der Erfindung liegt die Erkenntnis zugrunde, dass die Zugabe einer signifikanten Menge Polyvinylpyrrolidon die Stabilität der Percarbonsäuren erheblich erhöht. Es wurde gefunden, dass sich die Percarbonsäurekonzentration über einen langen Zeitraum praktisch nicht verändert, sodass sich eine Verwendbarkeit des Mittels über Monate oder Jahre ergibt. Der Effekt wird darauf zurückgeführt, dass Polyvinylpyrrolidon die Persäurefunktion komplexiert und dadurch die Abspaltung von Sauerstoff verhindert. Gleichzeitig wird die Aggressivität kurzkettiger Percarbonsäuren vermindert, so dass sie für die Anwendung auf der Haut infrage kommen.

Zudem ist Polyvinylpyrrolidon ein in der Pharmazie und Lebensmittelindustrie eingeführtes und vielfach verwandtes Produkt, das physiologisch unbedenklich ist, gut vertragen wird und zudem hautfreundlich ist. In Lebensmitteln wird es als Binde-und Fertigungsmittel eingesetzt, in Tabletten als Bindemittel und in der Wundbehandlung als Trägerstoff für Jod.

Polyvinylpyrrolidon, auch unter der Bezeichnung Povidon bekannt und vielfach mit PVP abgekürzt, ist ein sehr gut wasserlösliches Polymer mit einer amorphen Struktur. Es besitzt keinen Schmelzpunkt, sondern nur eine Glasübergangstemperatur, die je nach Polymerisationsgrad zwischen 110 und 180 °C liegt. Erfindungsgemäß können Polyvinylpyrrolidone aller Polymerisationsgrade eingesetzt werden.

Bevorzugt sind Polyvinylpyrrolidone eines mittleren Molekulargewichts im Bereich von 10.000 bis 360.000 Dalton. Als geeignet haben sich beispielsweise Polyvinylpyrrolidone mit mittleren Molekulargewichten (Gewichtsmittel) von 15.000 Dalton und insbesondere 40.000 Dalton (Povidon K 30) erwiesen.

Für erfindungsgemäße Zwecke kommen zusätzlich zur Hydroxypercabonsäure grundsätzlich alle Percarbonsäuren infrage, die in wässrige Lösung gebracht werden können. Dies sind beispielsweise gesättigte und ungesättigte aliphatische sowie aromatische Monocarbonsäuren mit bis zu 12 C-Atomen, etwa Peressigsäure, Perpropionsäure, Persorbinsäure und Perbenzoesäure, Hydroxycarbonsäuren mit bis zu 8 C-Atomen, wie Permilchsäure, Perzitronensäure, Peräpfelsäure und Perweinsäure, sowie beliebige Mischungen derselben. Die Persäure kann über mehrere Hydroxy-und/oder Säurefunktionen verfügen. Insbesondere multifunktionelle Percarbonsäuren, wie Perzitronensäure, haben sich für erfindungsgemäße Zwecke als sehr effizient erwiesen.

Der Gehalt an mit PVP stabilisierter Percarbonsäure (PVP plus Percarbonsäure) in dem erfindungsgemäßen Mittel beträgt zweckmäßigerweise 0,1 bis 50 Gew.-%, vorzugsweise 1,0 bis 25 Gew.-%. Das Gewichtsverhältnis PVP zu Percarbonsäure liegt dabei im Bereich von 1:10 bis 10:1, vorugsweise von 1:5 bis 5:1.

Naturgemäß ist die Wirksamkeit des Mittels umso größer, je höher die Percarbonsäurekonzentration ist, jedoch reichen viele Einsatzzwecke bereits Konzentrationen um 1 Gew.-% aus. In der Regel wird zunächst ein Konzentrat hergestellt, das über eine Percarbonsäurekonzentration zwischen 10 und 20 Gew.-% verfügt, dessen Konzentration anschließend durch Verdünnung mit Wasser oder einem Lösemittel herabgesetzt wird.

Als vorteilhaft hat sich erwiesen, in das Mittel mindestens zwei Percarbonsäuren einzubringen, da hierbei häufig synergistische Effekte beobachtet werden. So hat sich herausgestellt, dass unterschiedliche Percarbonsäuren sich für unterschiedliche Zwecke besonders eignen. Beispielsweise werden eine Reihe von Toxinen, sowohl Mikrotoxine als auch Endotoxine, die über eine Aldehyd- oder Ketofunktion verfügen, effektiv mittels Perbenzoesäure über eine Baeyer-Villiger-Oxidation zerstört. Perbenzoesäure ist gegen Aflatoxine einsetzbar und wirkt als Katalasehemmer. Peressigsäure wiederum ist geeignet, die Porenproteine von Bakterien effektiv anzugreifen, während Perzitronensäure sich als für den Abbau von Biofilmen besonders geeignet erwiesen hat. Persorbinsäure kann in Lipidschichten eindringen, auf diese Weise Zellmembranen durchdringen und Bakterien zu desaktivieren.

Das erfindungsgemäße Mittel kann zusätzlich zu der mit PVP stabilisierten Percarbonsäure in wässriger Lösung ein mit der Percarbonsäuren nicht reaktives Tensid enthalten, um die Netzfähigkeit zu erhöhen. In dieser Hinsicht bevorzugt sind Polysorbate in einer Menge von 0,5 bis 5 Gew.-%, beispielsweise Tween (Polysorbat 20) oder Natriumdodecylsulfat.

Das erfindungsgemäße Mittel kann übliche Zusatzstoffe enthalten, insbesondere Lösemittel, Sequestrierungsmittel, pH-Regulatoren, Korrosionsinhibitoren, weitere Persäurestabilisatoren, Komplexbildner, Entschäumungsmittel, Farb- und Duftstoffe, Verdickungsmittel und Pflegemittel. Es versteht sich, dass alle diese Zusatzstoffe gegenüber Percarbonsäuren stabil sein müssen und auch den Zerfall der Percarbonsäuren nicht fördern. Korrosionsinhibitoren sind insbesondere dann sinnvoll, wenn die Zusammensetzung zur Behandlung von Metalloberflächen eingesetzt wird. Als Korrosionsinhibitoren können beispielsweise Alkalimetallphosphate, bevorzugt Kaliumsphosphate eingesetzt werden, etwa Dikaliumhydrogenorthophosphat.

Als Lösemittel dient in erster Linie Wasser. Das Mittel kann zudem organische Lösungsmittel enthalten, insbesondere Ethanol und Isopropanol. Alkohol weisen den Vorteil auf, dass sie einen zusätzlichen bakteriziden und fungiziden Effekt aufweisen, wodurch die keimtötende Wirkung der erfindungsgemäßen Zusammensetzung verstärkt wird.

Für die Verwendung als Desinfektionsmittel von Körperoberflächen, also Haut und Schleimhaut, ist das Mittel vorzugsweise als Spray, Gel, Creme oder Lotion formuliert. Auch hier gilt, dass die Formulierungshilfsstoffe oxidationsstabil sein müssen.

Der pH-Wert der wässrigen Lösung sollte im sauren Bereich liegen. Percarbonsäuren neigen in alkalischem Milieu zur Zersetzung. Typischerweise liegt der pH-Wert der für Anwendung der verdünnten Lösung zwischen 0 und 7, insbesondere zwischen 3 und 6. Gegebenenfalls kann der pH-Wert durch Einsatz von pH-Regulatoren eingestellt werden.

Das erfindungsgemäße Mittel hat sich als hochwirksam gegen Bakterien-, Virus- und Pilzinfektionen erwiesen. Darüber hinaus ist es auch geeignet, Stoffwechselprodukte von Mikroorganismen zu zerstören. Dies gilt sowohl für toxische Stoffwechselprodukte (Biotoxine) von Bakterien als auch für Adhäsine, also bakterielle Produkte, die die Anhaftung und das Wachstum von Bakterien an den Körper oder an Wunden fördern. Entsprechend ist das erfindungsgemäße Mittel als Desinfektions- und Reinigungsmittel für die Haut und Schleimhaut geeignet.

Bei der Bekämpfung von Mikroorganismen bewirkt die Freisetzung von Sauerstoffradikalen eine schnelle Oxidation der Außenhülle/Zellmembran. Auch Enzyme und andere Proteine sowie Nukleinsäuren werden durch die Percarbonsäuren angegriffen und geschädigt durch die daraus folgende Blockierung der Stoffwechselwege (Energiestoffwechsel, Proteinbiosynthese) tritt rasch der Zelltod ein daneben werden durch die erfindungsgemäße Zusammensetzung auch Biotoxine - Aflatoxine, Endotoxine, Mykotoxine und Allergene - zerstört.

Die Percarbonsäuren des erfindungsgemäßen Mittels sind in der Lage, die Tertiärstruktur von Proteinen dass diese ihre Eigenschaften verlieren. So werden Disulfidbrücken durch Oxidation aufgebrochen. Thiolgruppen werden zu Sulfonsäuren oxidiert.

Die erfindungsgemäßen Zusammensetzungen sind auch in der Lage, die Stoffwechselzentren von Mikroorganismen durch Oxidation der Enzyme, welche meist Schwermetalle wie beispielsweise Kupfer enthalten, zu deaktivieren. Viren können dadurch deaktiviert werden, dass die Kapsidproteine und Hüllproteine oxidiert werden. Dies führt zu einer Desintegration des Nukleokapsids oder zu einer Veränderung der Kapsidoberfläche, die Aktivität des Virus zerstört.

Bei der Oxidation und der damit verbundenen Zerstörung von schädlichen Mikroorganismen wandelt sich die Percarbonsäuren wieder zur Carbonsäure selbst um, sodass letztlich nur vergleichsweise harmlose Produkte entstehen.

Das erfindungsgemäße Mittel kann mit Vorteil in der Landwirtschaft eingesetzt werden, beispielsweise im Pflanzenschutz, bei der Pflanzenstärkung, zur Desinfektion und Reinigung von Stallungen, zur Abluftbehandlung und zur Desinfektion von landwirtschaftlichen Lagerräumen. Im Zusammenhang wird ausdrücklich auf die in der WO 2006/125 657 A2 genannten Anwendungen verwiesen.

Das erfindungsgemäße Mittel kann ferner zur Reinigung und Desinfektion von Wunden dienen wie auch zur Behandlung von Fuß-und Nagelpilz, gegen Warzen und als Spülmittel bei der Mund-und Zahnhygiene. Es kann weiterhin bei Tieren zur Reinigung und Desinfektion wie auch zur Behandlung von Haut-, Fell- und Klauen/Huferkrankungen eingesetzt werden.

Das erfindungsgemäße Mittel kann analog zu den in der WO 2006/125 657 A2 beschriebenen Prinzipien hergestellt werden. Als Ausgangsmaterialien kommen neben den jeweiligen Carbonsäuren auch deren Anhydride und üblichen Salze in Frage. Nachstehende Beispiele erläutern das Verfahren.

### Beispiel 1

In 90 g einer Wasserstoffperoxidlösung (20%) in Wasser werden zunächst 5 g Zitronensäure in Gegenwart einer katalytischen Menge Phosphorsäure bei Raumtemperatur zu Perzitronensäure umgesetzt. Nach der Gleichgewichtseinstellung wird die Mischung mit 5 g PVP K30 versetzt. Diese Mischung wird dann mit der zehnfachen Menge Wasser auf Anwendungsstärke verdünnt. Die Gleichgewichtseinstellung der Mischung kann einige Tage dauern. Ein Überschuss an Wasserstoffperoxid verschiebt das Gleichgewicht hin zur Percarbonsäure Zur Beschleunigung kann der Mischung eine geringe Menge einer starken Säure, beispielsweise Methansulfonsäure, Phosphorsäure oder Schwefelsäure als Katalysator beigemischt werden.

Die so erhaltene Mischung ist zur Behandlung und Desinfektion von Hautwunden geeignet. Zur Herstellung eines Gels kann ein übliches Verdickungsmittel zugesetzt werden, etwa ein Acrylat.

### Beispiel 2

Ein gegen Warzen aktives Mittel wird wie folgt hergestellt:
90 g einer Wasserstoffperoxidlösung in Wasser (35%) werden vorgelegt und bei Raumtemperatur mit 1 g Benzoesäure, 3 g Zitronensäure und 1 g Natriumdiacetat versetzt. Die Mischung wird bis zur Einstellung des Reaktionsgleichgewichts gerührt und anschließend mit 5 g PVP K30 versetzt. Nach weiterem Rühren werden 900 ml deionisiertes Wasser, danach weitere 20 g PVP K30 zugegeben. Weitere Verdünnung mit 1000 ml deionisiertem Wasser ergibt das fertige Mittel.

## Patentansprüche

1. Mittel zum Desinfizieren von Oberflächen und Zerstören von bakteriellen Stoffwechselprodukten, enthaltend wenigstens eine Percarbonsäure zusammen mit einem Stabilisierungsmittel in wässriger Lösung, **dadurch gekennzeichnet, dass** das Stabilisierungsmittel Polyvinylpyrrolidon (PVP) ist, wobei die Percarbonsäure eine Hydroxypercarbonsäure ist.

2. Mittel nach Anspruch 1, **dadurch gekennzeichnet, dass** das PVP ein mittleres Molekulargewicht von 10.000 bis 360.000 Dalton hat.

3. Mittel nach Anspruch 2, **dadurch gekennzeichnet, dass** das PVP ein mittleres Molekulargewicht von 40.000 Dalton hat.

4. Mittel nach einem der Ansprüche 1 bis 3, **dadurch gekennzeichnet, dass** die Percarbonsäure, Permilchsäure, Perzitronensäure, Peräpfelsäure oder Perweinsäure ist.

5. Mittel nach einem der vorstehenden Ansprüche, **dadurch gekennzeichnet, dass** das Gewichtsverhältnis Percarbonsäure zu PVP im Bereich von 1:10 bis 10:1 liegt.

6. Mittel nach einem der vorstehenden Ansprüche, **dadurch gekennzeichnet, dass** die wässrige Lösung 0,1 bis 50 Gew.-%, vorzugsweise 1 bis 25 Gew.-% mit PVP stabilisierte Percarbonsäure enthält.

7. Mittel nach einem der vorstehenden Ansprüche, **dadurch gekennzeichnet, dass** es weiterhin ein oxidationsstabiles Tensid enthält.

8. Mittel nach Anspruch 7, **dadurch gekennzeichnet, dass** es 0, 5 bis 5 Gew.-% Polysorbat enthält.

9. Mittel nach einem der vorstehenden Ansprüche, **dadurch gekennzeichnet, dass** das Mittel Lösemittel, Sequestrierungsmittel, pH-Regulatoren, Korrosionsinhibitoren, Stabilisierungsmittel für Persäuren, Komplexbildner, Entscheidungsmittel, Farbstoffe und/oder Duftstoffe enthält.

10. Mittel nach einem der vorstehenden Ansprüche, **dadurch gekennzeichnet, dass** es einen pH-Wert von 0 bis 7, vorzugsweise von 3 bis 6 aufweist.

11. Nicht therapeutische Verwendung des Mittels nach einem der vorstehenden Ansprüche zum Entkeimen von Oberflächen.

12. Nicht therapeutische Verwendung des Mittels nach einem der Ansprüche 1 bis 9 zur Zerstörung von bakteriellen Stoffwechselprodukten.

13. Verwendung des Mittels nach einem der Ansprüche 1 bis 9 zur Stalldesinfektion und im Pflanzenschutz.

14. Zusammensetzung in Form eines Sprays oder Gels, enthaltend das Mittel nach einem der Ansprüche 1 bis 8.

15. Verwendung von Polyvinylpyrrolidon zur Stabilisierung von Hydroxypercarbonsäuren in wässriger Lösung.

## Claims

1. Agent for the disinfection of surfaces and the destruction of bacterial metabolic products, said agent containing at least one percarboxylic acid together with a stabilizing agent in aqueous solution, **characterized in that** polyvinylpyrrolidone (PVP) is used as stabilizing agent, the agent comprising a hydroxypercarboxylic acid.

2. Agent according to claim 1, **characterized in that** the PVP has an average molecular weight ranging between 10,000 and 360,000 daltons.

3. Agent according to claim 2, **characterized in that** the PVP has an average molecular weight of 40,000 daltons.

4. Agent according to one of claims 1 to 3, **characterized in that** the percarboxylic acid is perlactic acid, percitric acid, permalic acid or pertartaric acid.

5. Agent according to any one of the preceding claims, **characterized in that** the weight ratio of percarboxylic acid with respect to PVP is in the range between 1:10 and 10:1.

6. Agent according to any one of the preceding claims, **characterized in that** the aqueous solution contains 0.1 to 50% w/w, preferably 1 to 25% w/w, of percarboxylic acid stabilized with PVP.

7. Agent according to any one of the preceding claims, **characterized in that** it also contains an oxidation-stable surfactant.

8. Agent according to claim 7, **characterized in that** it contains 0.5 to 5% w/w of polysorbate.

9. Agent according to any one of the preceding claims, **characterized in that** the agent contains solvents, sequestrants, pH regulators, corrosion inhibitors, stabilizing agents for peracids, complexing agents, anti-foaming agents, colorants and/or odorants.

10. Agent according to any one of the preceding claims, **characterized in that** said agent has a pH value of 0 to 7, preferably 3 to 6.

11. Non-therapeutic use of the agent in accordance with any one of claims 1 to 9 for the sterilization of surfaces.

12. Non-therapeutic use of the agent according to any one of claims 1 to 9 for the destruction of bacterial metabolic products.

13. Use of the agent according to any one of the claims 1 to 9 for the disinfection of stables and for plant/crop protection purposes.

14. Composition in the form of a spray or gel containing the agent according to any one of claims 1 to 8.

15. Use of polyvinylpyrrolidone for stabilizing hydoxypercarboxylic acids in aqueous solution.

## Revendications

1. Agent pour la désinfection de surfaces et la destruction de métabolites bactériens, contenant au moins un acide percarboxylique conjointement avec un agent stabilisant en solution aqueuse, **caractérisé en ce que** l'agent stabilisant est la polyvinylpyrrolidone (PVP), l'acide percarboxylique étant un acide hydroxypercarboxylique.

2. Agent selon la revendication 1, **caractérisé en ce que** la PVP a un poids moléculaire moyen de 10 000 à 360 000 daltons.

3. Agent selon la revendication 2, **caractérisé en ce que** la PVP a un poids moléculaire moyen de 40 000 daltons.

4. Agent selon l'une quelconque des revendications 1 à 3, **caractérisé en ce que** l'acide percarboxylique est l'acide perlactique, l'acide percitrique, l'acide permalique ou l'acide pertartrique.

5. Agent selon l'une quelconque des revendications précédentes, **caractérisé en ce que** le rapport en poids de l'acide percarboxylique à la PVP se situe dans la plage allant de 1:10 à 10:1.

6. Agent selon l'une quelconque des revendications précédentes, **caractérisé en ce que** la solution aqueuse contient 0,1 à 50 % en poids, de préférence 1 à 25 % en poids d'acide percarboxylique stabilisé avec de la PVP.

7. Agent selon l'une quelconque des revendications précédentes, **caractérisé en ce qu'**il contient en outre un tensioactif stable à l'oxydation.

8. Agent selon la revendication 7, **caractérisé en ce qu'**il contient 0,5 à 5 % en poids de polysorbate.

9. Agent selon l'une quelconque des revendications précédentes, **caractérisé en ce que** l'agent contient des solvants, des agents séquestrants, des régulateurs de pH, des inhibiteurs de corrosion, des agents stabilisants pour les peracides, des agents complexants, des agents de décision, des colorants et/ou des parfums.

10. Agent selon l'une quelconque des revendications précédentes, **caractérisé en ce qu'**il présente un pH de 0 à 7, de préférence de 3 à 6.

11. Utilisation non thérapeutique de l'agent selon l'une quelconque des revendications précédentes pour la désinfection de surfaces.

12. Utilisation non thérapeutique de l'agent selon l'une quelconque des revendications 1 à 9 pour la destruction de métabolites bactériens.

13. Utilisation de l'agent selon l'une quelconque des revendications 1 à 9 pour la désinfection d'étables et dans la protection de plantes.

14. Composition sous la forme d'une pulvérisation ou d'un gel, contenant l'agent selon l'une quelconque des revendications 1 à 8.

15. Utilisation de la polyvinylpyrrolidone pour la stabilisation d'acides hydroxypercarboxyliques en solution aqueuse.
